# FASCICULE DE BREVET EUROPEEN

(11) **EP 0 704 001 B1**
(45) Date de publication et mention de la délivrance du brevet: **26.02.1997**
(21) Numéro de dépôt: 94919707.3
(22) Date de dépôt: 14.06.1994
(51) Int. Cl.: D04H 13/00, D04H 1/42, A61F 13/15

(54) **MATERIAU NON-TISSE COMPOSITE, PROCEDE DE FABRICATION ET SON APPLICATION A TOUT ARTICLE D'HYGIENE ABSORBANT**
VERBUNDVLIESSTOFF, VERFAHREN ZU DESSEN HERSTELLUNG UND DESSEN VERWENDUNG IN ALLEN ABSORBIERENDEN HYGIENEARTIKELN
COMPOSITE NONWOVEN MATERIAL, METHOD FOR PRODUCING SAME AND USE THEREOF IN ANY ABSORBENT SANITARY ARTICLE

(30) Priorité: 16.06.1993 FR 9307239
(43) Date de publication de la demande: 03.04.1996
(73) Titulaire: PEAUDOUCE, 59126 Linselles (FR)
(72) Inventeur: KOCZAB, Jean-Pierre, F-59910 Bondues (FR)
(74) Mandataire: Casalonga, Axel
(86) Numéro de dépôt international: FR9400710
(87) Numéro de publication internationale: WO9429506

(56) Documents cités:
- EP-A- 0 516 877
- EP-A- 0 532 005
- GB-A- 2 085 358
- US-A- 5 143 779

## Description

L'invention concerne, d'une manière générale, un nouveau matériau non-tissé, qui lorsqu'il est utilisé comme voile ou feuille de surface ou en complément de la feuille de surface comme bande dans la zone d'entre-jambes dans un article d'hygiène absorbant, tel qu'une couche-culotte ou garniture pour incontinents, permet une meilleure isolation de la peau de l'utilisateur de la partie absorbante de l'article d'hygiène. En particulier, ce nouveau matériau favorise le temps de transpercement par les liquides corporels, la résistance au remouillage, et ne peluche pas.

D'une manière générale, les articles d'hygiène absorbants, tels que des couche-culottes et garnitures pour incontinents, comprennent une couche externe en matériau imperméable aux liquides, un coussin en matériau absorbant et un voile ou feuille de surface perméable aux liquides corporels, tels que l'urine, de dimension et de forme analogues à celles de la couche externe imperméable de l'article. Cette feuille de surface perméable aux liquides corporels a pour but d'isoler la peau du coussin absorbant humidifié. Par conséquent, la feuille de surface doit avoir un degré de douceur convenable et assurer une isolation voulue entre la peau et le coussin absorbant. Le coussin absorbant a pour fonction d'absorber les liquides et par conséquent doit présenter une vitesse d'absorption importante ainsi qu'une grande capacité d'absorption. Un coussin absorbant particulièrement efficace est décrit dans le document EP-A-0 232729. Ce coussin ou matelas absorbant se compose d'une nappe de fibres absorbantes longues doublée sur ses faces d'une couche de ouate de cellulose. La nappe doublée par les couches de ouate est aiguilletée depuis les deux faces.

Dans les articles d'hygiène absorbants de tels matelas ou coussins absorbants sont recouverts par une feuille de surface ou par une bande dans la zone d'entre-jambes, généralement en matériau non-tissé, qui a pour but d'isoler la peau du coussin absorbant et qui doit assurer un contact agréable avec la peau et l'isolation voulue avec le coussin absorbant. Ces voiles ou feuilles de surface et bandes de zone d'entre-jambes doivent présenter comme propriétés essentielles un contact agréable avec la peau, une vitesse de traversée par les liquides corporels importante, une bonne résistance au remouillage et ne pas pelucher.

Le document FR-A-2 588 285 décrit un textile non-tissé multi-couches ayant au moins deux couches de voile non-tissé, l'une des couches étant formée de fibres de section transversale bilobée et l'autre couche étant formée de fibres de section transversale trilobée. Chaque couche de voile est de préférence obtenue par la technique de liaison au filage (Spun bonded) et les deux couches de voile sont réunies pour former le non-tissé multicouche par liaison thermique en des zones compactées et discontinues.

Le document WO 87/07117 décrit un article d'hygiène absorbant comprenant un corps absorbant entouré d'une enveloppe. Cette enveloppe ou voile de surface est constituée de deux couches en matériau non-tissé. La première couche de matériau non-tissé, en contact avec la peau de l'utilisateur, est constituée d'une mince couche de tissus fibreux lié au filage en un matériau hydrophobique, et la seconde couche en contact avec le corps absorbant est une couche fibreuse hydrophobique de tissu de fibres liée par fusion, de construction similaire à la première couche. Ces deux couches de voile de surface ne sont pas liées entre elles dans la zone destinée à venir en contact avec le corps de l'utilisateur.

Le document WO 88/05269 concerne un voile de surface pour un article absorbant jetable composé d'au moins deux couches de matériau non-tissé qui peuvent être identiques ou différentes et qui sont réunies par des lignes d'adhésif formant un motif ouvert.

On a également récemment mis au point un nouveau matériau non-tissé composite comprenant au moins une première couche constituée par un non-tissé (de préférence lié au filage) et, sur cette première couche une nappe de fibres de type cardé, la nappe de fibres de type cardé étant liée à la couche de base par aiguilletage.

Un autre matériau non-tissé composite récemment mis au point comprend une première couche en un non-tissé (de préférence lié au filage), une nappe de fibres de type cardé et une seconde couche en un non-tissé (de préférence lié au filage) de grammage inférieur à la première couche de non-tissé, la nappe de fibres de type cardé étant disposée entre la première et la deuxième couche de non-tissé, et l'ensemble étant lié par aiguilletage.

Ces matériaux présentent d'excellents temps de traversée par les fluides corporels et une excellente résistance au remouillage. Toutefois, ces matériaux ont une tendance prononcée au peluchage lorsqu'ils sont utilisés comme voile de surface ou bande d'entre-jambes dans des articles d'hygiène absorbants, tels que des couches-culottes.

Le document WO 91/14414 décrit un non-tissé composite comprenant une première couche constituée d'au moins 75 % en poids de fibres thermoplastiques hydrophobes et une seconde couche constituée d'un mélange d'environ 20 à 70 % en poids de fibres thermoplastiques hydrophobes et d'environ 30 à 80 % en poids de fibres naturelles hydrophiles, les couches étant fixées entre elles par des parties fondues. Les couches peuvent être formées de voiles cardés ou non et les fibres thermoplastiques hydrophobes peuvent être choisies parmi la fibres de polyoléfine, polyesters ou bi-composants.

La présente invention a donc pour but de fournir un matériau non-tissé composite qui, lorsqu'il est utilisé comme feuille de surface ou bande d'entre-jambes dans un article d'hygiène absorbant, présente une excellente vitesse de traversée par les fluides corporels, une bonne résistance au remouillage et ne peluche pas.

L'invention a également pour but de fournir un procédé de fabrication d'un tel matériau non-tissé composite.

La présente invention a enfin pour but de fournir un article d'hygiène absorbant comportant une feuille de surface ou une bande de zone d'entre-jambes formé d'un tel matériau non-tissé composite.

Selon la présente invention, on réalise un matériau non-tissé composite perméable aux liquides corporels caractérisé en ce qu'il comprend au moins une première couche d'un voile cardé de fibres thermoplastiques hydrophobes, ces fibres étant partiellement désorientées dans au moins une partie de l'épaisseur du voile pour qu'une partie de ces fibres aient une orientation angulaire par rapport au plan principal des fibres du voile cardé et une seconde couche d'un voile de non-tissé de type non cardé, de fibres thermoplastiques, hydrophobes, les fibres d'au moins une des couches étant choisies parmi les fibres à bas point de fusion, les fibres bi-composants, les mélanges de telles fibres avec des fibres à point de fusion élevé, les fibres mélangées avec un liant tel qu'une colle réactivable à chaud ou une poudre à bas point de fusion, et les mélanges de telles fibres avec des fibres à point de fusion élevé, ou au moins une des couches est formée par un voile dont une face est enduite d'un liant, les couches étant liées entre elles uniquement par thermofusion.

L'invention fournit également un procédé de fabrication d'un matériau non-tissé composite comprenant les étapes consistant à :
- former un voile cardé, de fibres thermoplastiques, hydrophobes;
- désorienter partiellement les fibres du voile cardé pour obtenir dans au moins une partie de l'épaisseur du voile cardé une orientation angulaire d'une partie des fibres par rapport au plan principal des fibres du voile cardé;
- former un voile de non-tissé de type non-cardé, de fibres thermoplastiques, hydrophobes;
- superposer le voile cardé à fibres partiellement désorientées et le voile en non-tissé de type non-cardé, pour obtenir une première couche du voile à fibres partiellement désorientées sur une seconde couche du voile en non-tissé de type non-cardé, les fibres d'au moins une desdites couches étant choisies parmi les fibres à bas point de fusion, les fibres bi-composants, les mélanges de telles fibres avec des fibres à point de fusion élevé, et les fibres mélangées avec un liant tel qu'une colle réactivable à chaud ou une poudre à bas point de fusion; et
- lier entre elles les couches par thermofusion, par exemple par réactivation au moyen d'air chaud traversant les couches.

Une autre réalisation du procédé selon l'invention comprend les étapes consistant à :
- former un voile cardé de fibres thermoplastiques, hydrophobes;
- désorienter partiellement les fibres du voile cardé pour obtenir dans au moins une partie de l'épaisseur du voile cardé une orientation angulaire d'une partie des fibres par rapport au plan principal des fibres du voile cardé;
- former un voile en non-tissé de type non-cardé, de fibres thermoplastiques, hydrophobes;
- enduire une des faces d'un desdits voiles avec un liant, par exemple une colle réactivable à chaud;
- superposer le voile cardé à fibres partiellement désorientées et le voile en non-tissé de type non-cardé pour obtenir une première couche du voile cardé et une deuxième couche de non-tissé de type non-cardé de telle sorte que ladite face enduite de liant forme une interface entre lesdites couches; et
- lier les couches entre elles par thermofusion.

Selon la présente invention on réalise également un article d'hygiène absorbant tel qu'une couche-culotte qui comprend une couche externe en matériau imperméable aux liquides corporels, un coussin absorbant perméable aux liquides corporels et sur ce coussin absorbant soit une feuille de surface, soit une feuille de surface et une bande de zone d'entre-jambes, la feuille de surface, lorsqu'elle est utilisée seule, étant constituée du matériau non-tissé composite selon l'invention et la bande de zone d'entre-jambes étant constituée du matériau non-tissé composite selon l'invention et la feuille de surface, d'un non-tissé, de préférence hydrophobe, lorsqu'on utilise conjointement une bande de zone d'entre-jambes et une feuille de surface. Dans l'article d'hygiène la feuille de surface ou la bande de zone d'entre-jambes constitué par le matériau composite selon l'invention, est disposée de telle sorte que la couche en voile cardé soit en contact direct avec la surface interne du coussin absorbant, et par conséquent la couche en non-tissé de type non-cardé soit en contact direct avec la peau de l'utilisateur ou en contact avec la surface externe de la feuille de surface dans le cas où l'article comporte une feuille de surface en non-tissé.

Dans une réalisation recommandée l'invention fournit un article d'hygiène absorbant tel qu'une couche-culotte, qui comprend une couche externe en matériau imperméable aux liquides corporels, un coussin absorbant perméable aux liquides corporels fixé à la couche externe, la couche externe et le coussin absorbant comprenant des parties d'extrémité opposées élargies réunies par une zone d'entre-jambes plus étroite, une feuille de surface, de préférence hydrophobe, une bande de zone d'entre-jambes, perméable aux liquides corporels, disposée entre le coussin et la feuille de surface et de largeur analogue à la zone d'entre-jambes du coussin et de longueur au moins égale à celle du coussin, cette bande de zone d'entre-jambes étant constituée du matériau non-tissé composite selon l'invention, la couche en voile cardé étant directement en contact avec la surface interne du coussin absorbant.

Dans une réalisation particulièrement recommandée, la feuille de surface comporte une découpe longitudinale médiane formant une ouverture, de préférence de forme oblongue.

Dans une autre réalisation une bande intermédiaire, en non-tissé, de dimension analogue à celle de la bande de zone d'entre-jambes est disposée directement sur la bande de zone d'entre-jambes, en dessous de la feuille de surface.

La bande de zone d'entre-jambes est en général liée au coussin absorbant par tout moyen approprié, et en particulier par collage sur le bord du coussin absorbant.

La suite de la description se réfère aux figures annexées qui représentent respectivement :
figure 1, une vue schématique en coupe d'un matériau non-tissé composite selon l'invention;
figure 2, une vue de dessus, en partie arrachée, d'un article d'hygiène absorbant tel qu'une couche-culotte comportant une bande de zone d'entre-jambes constituée du matériau non-tissé composite selon l'invention et une bande intermédiaire recouvrant la bande de zone d'entre-jambes;
figure 3, une vue en coupe faite suivant la ligne m-m de la figure 2, et
figure 4, une vue en coupe d'une couche-culotte analogue à celle de la figure 3, mais dans laquelle on a supprimé la bande intermédiaire.

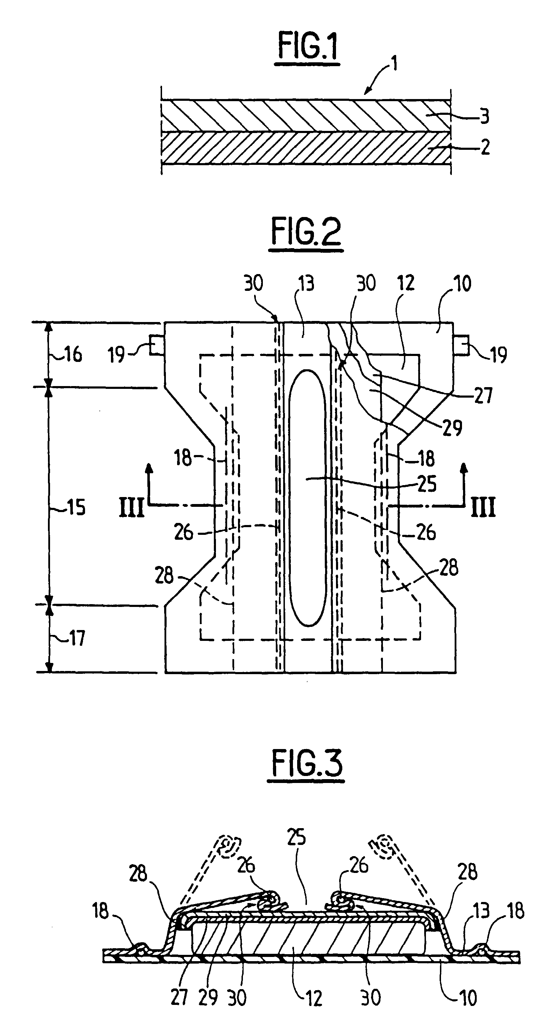

En se référant à la figure 1 on a représenté schématiquement en coupe un matériau non-tissé composite 1 selon l'invention. Ce matériau comprend une première couche 2 constituée d'un voile cardé de fibres thermoplastiques, hydrophobes, ces fibres étant désorientées dans au moins une partie de l'épaisseur du voile pour qu'au moins une partie des fibres aient une orientation angulaire par rapport au plan principal des fibres du voile cardé et une seconde couche 3 constituée d'un voile en non-tissé de type non-cardé, de fibres thermoplastiques, hydrophobes.

Dans la présente invention on désigne par l'expression "voile cardé" un voile en non-tissé obtenu par la technique classique du cardage.

Les voiles obtenus par cardage ont pour caractéristique que les fibres sont sensiblement toutes orientées suivant un même plan principal, généralement le plan du défilement de la machine.

Selon la présente invention, les fibres du voile cardé sont en partie désorientées par rapport au plan principal des fibres du voile pour qu'au moins dans une partie de l'épaisseur du voile une certaine quantité de fibres soient orientées angulairement par rapport à ce plan principal des fibres. De préférence, les fibres désorientées font un angle d'au moins 35° par rapport au plan principal des fibres. De préférence les fibres désorientées du voile cardé font un angle compris entre 45 et 90° par rapport au plan principal des fibres.

Une telle désorientation des fibres peut s'obtenir directement lors de la fabrication du voile cardé par l'utilisation d'une pseudo-carde. Ces pseudo-cardes sont des appareillages connus en eux-mêmes et sont vendus dans le commerce, par exemple par la Société FEHRER.

Le taux de fibres désorientées du voile cardé est généralement d'au moins 20 % en poids, de préférence de 25 % à 85 % en poids par rapport au poids total de fibres du voile.

Cette désorientation d'une partie des fibres du voile cardé augmente la vitesse de transpercement du matériau composite par les liquides.

On peut utiliser pour les voiles cardés et en non-tissé de type non-cardé toutes fibres textiles thermoplastiques et hydrophobes telles que des fibres à point de fusion élevé, des fibres à bas point de fusion, des fibres bi-composants, des fibres mélangées avec un liant, tel qu'une colle réactivable à chaud ou une poudre de polymère à bas point de fusion, et des mélanges de fibres à point de fusion élevé avec des fibres à bas point de fusion, et des fibres bi-composants.

Dans la présente invention les fibres thermoplastiques hydrophobes peuvent être classiquement revêtues avec un fini de filage qui contient des lubrifiants, des agents antistatiques et mouillants. Les agents mouillants favorisent le passage initial des liquides à travers le matériau composite.

Dans la présente invention on désigne par "fibres à point de fusion élevé" des fibres dont la composition est telle qu'elles ne se lient pas sous l'effet d'un chauffage modéré (thermofusion), par exemple par réactivation à l'air chaud. De telles fibres sont par exemples des fibres de polyester ou de polyamide.

On désigne par "fibres à bas point de fusion" des fibres dont la composition est telle qu'elles se ramollissent ou fondent superficiellement sous l'effet d'un chauffage modéré et ainsi se lient entre elles (thermofusion). De telles fibres sont par exemple, des fibres de polyoléfine comme les fibres de polyéthylène, polypropylène et de copolymères d'éthylène-propylène.

Les fibres bi-composants sont des fibres comprenant une âme en un polymère à point de fusion relativement élevé enveloppée par un autre polymère de point de fusion relativement bas permettant une fusion ou un ramollissement de la surface de la fibre sous l'effet d'un chauffage modéré, tel qu'un chauffage à l'air chaud. De telles fibres sont bien connues dans la technique. Un exemple de fibres bi-composants est une fibre comprenant une âme de polyester enveloppée de polyoléfine, par exemple de polyéthylène.

On désigne par "liant" tout produit qui mélangé aux fibres confère à celles-ci la possibilité de se lier entre elles lors d'un chauffage modéré (thermofusion), par exemple par calandrage ou soufflage d'air chaud. Parmi ces liants on peut citer les colles réactivables à chaud et les poudres à bas point de fusion.

Les colles réactivables à chaud sont bien connues dans la technique, et un exemple d'une telle colle est une solution aqueuse de type EVA, EBA ou acrylique.

De même les poudres à bas point de fusion utilisables dans la présente invention sont bien connues dans la technique et sont généralement des poudres de polymères à bas point de fusion, telles que, par exemple une poudre de polyéthylène.

Les voiles cardés et en non-tissé de type non-cardé du matériau composite de l'invention peuvent être chacun constitués de fibres de même nature ou de natures différentes, et ayant des deniers identiques ou différents.

Le voile cardé a de préférence un grammage compris entre 20 et 60 g/m², en particulier de 50 g/m², et le voile de type non-cardé a, de préférence un grammage inférieur à 20 g/m², par exemple de 15 g/m².

Selon l'invention au moins une des couches du composite est constituée par des fibres thermoplastiques, hydrophobes à bas point de fusion, bi-composants, mélangées à un liant ou par un mélange de ces fibres avec des fibres thermoplastiques, hydrophobes à point de fusion élevé. De préférence, le voile en non-tissé de type non-cardé est constitué de fibres à bas point de fusion, telles que des fibres de polypropylène, et le voile cardé est constitué d'un mélange de fibres à point de fusion élevé et de fibres à bas point de fusion ou de fibres bi-composants, ou encore un des voiles est enduit sur une de ses faces avec une colle réactivable à chaud.

De préférence, encore, chacun des voiles est constitué par un mélange de fibres à point de fusion élevé, telles que des fibres de polyester, avec des fibres à bas point de fusion telles que des fibres de polyoléfine, par exemple de polyéthylène, ou des fibres bi-composants, ou encore de fibres mélangées avec un liant tel qu'une colle réactivable à chaud ou poudre à bas point de fusion. La proportion de fibres à bas point de fusion ou à bi-composants, dans le cas d'un voile constitué par un mélange de fibres, ou encore la quantité de liant mélangé aux fibres ou déposé sur une des faces d'un ou des voiles est généralement comprise entre 5 et 30% en poids par rapport au poids total du voile.

Les couches formées des voiles cardé 2 et en non-tissé de type non-cardé 3 sont liées par thermofusion, c'est-à-dire par un chauffage modéré des couches, par tout moyen connu dans la technique, tel que soufflage d'air chaud, calandrage, liaison aux ultrasons et par rayonnement infra-rouge. La température et la durée d'application de chaleur doivent être suffisantes pour assurer une liaison des fibres par ramollissement ou fusion superficielle des fibres à bas point de fusion, du composant à bas point de fusion des fibres bi-composants ou du liant, mais insuffisantes pour provoquer une dégradation des fibres des couches. De préférence, la température de liaison est comprise entre 120 et 150°C.

On recommande particulièrement la liaison par soufflage d'air et par calandrage, par exemple, par passage des couches du composite dans une calandre de contre-collage du type STORCK ayant un cylindre à une température de 120-150°C. Ce deuxième procédé convient particulièrement lorsque l'un des voiles du composite comporte une face enduite d'un liant. Bien évidemment dans ce cas le voile dont une face est enduite de liant est liée par cette face à l'autre voile du composite.

Le voile cardé formant la première couche du composite peut lui-même comporter plusieurs couches de voile cardé, liées entre elles par thermofusion, et par conséquent un nombre approprié de couches du voile cardé composite comprendront des fibres à bas point de fusion, des fibres bi-composants, des fibres mélangées à un liant ou auront une face enduite d'un liant pour permettre la liaison par thermofusion des couches du voile cardé.

Les couches du voile composite cardé peuvent être constituées par des fibres de nature identique ou différente et avoir des deniers identiques ou différents.

Le procédé de fabrication selon l'invention d'un matériau non-tissé composite comprend :
- la formation d'un voile cardé en fibres thermoplastiques, hydrophobes;
- la désorientation des fibres du voile cardé pour obtenir dans au moins une partie de l'épaisseur du voile une orientation angulaire d'une partie des fibres par rapport à l'orientation principale des fibres du voile cardé;
- l'application ou dépôt du voile cardé sur un voile en non-tissé de type non-cardé en fibres thermoplastiques, hydrophobes; et
- la liaison, par thermofusion, des voiles pour former un matériau non-tissé composite comprenant une première couche d'un voile cardé à fibres partiellement désorientées et une seconde couche d'un voile en non-tissé de type non-cardé, les première et seconde couches étant liées uniquement par thermofusion.

Dans un procédé recommandé, le voile cardé à fibres partiellement désorientées est obtenu au moyen d'une pseudo-carde, par exemple de type K12 ou K21 de la société FEHRER. L'utilisation d'une pseudo-carde pour la fabrication du voile cardé permet en une seule étape de former le voile cardé et de désorienter une partie des fibres dans une partie de l'épaisseur du voile.

On peut également procéder en deux étapes distinctes, en formant d'abord le voile cardé au moyen d'une carde puis en désorientant une partie des fibres par tout moyen mécanique approprié.

### Exemples de réalisation de matériaux non-tissés composites selon l'invention.

### Exemple 1

On forme au moyen d'une pseudo-carde un voile cardé à fibres partiellement désorientées comprenant un mélange de fibres de polyester de 6 décitex (deniers) avec des fibres de polyéthylène de 3,3 décitex (deniers) et ayant un grammage de 50 g/m² et on le superpose sur un voile en non-tissé de type lié au filage (Spun) en fibres de polypropylène, ayant un grammage de 15 g/m2. La proportion de fibres de polyéthylène dans le voile cardé est de 5 à 30% en poids par rapport au poids total du voile cardé.

On soumet l'ensemble des deux voiles à un chauffage modéré, par traversée d'air chaud à 120-150°C, pour former un matériau non-tissé composite comprenant une première couche d'un voile cardé et une deuxième couche d'un voile en non-tissé de type lié au filage (Spun), les première et seconde couche étant liées uniquement par thermofusion.

Ce matériau présente un excellent temps de traversée par les liquides, une bonne résistance au remouillage et ne peluche pas.

D'autre part, lorsqu'il est utilisé comme voile de surface ou en complément d'un voile de surface, comme bande de zone d'entre-jambes, en plaçant la seconde couche en voile de type lié au filage vers l'intérieur, c'est-à-dire en contact avec la peau de l'utilisateur ou de la surface externe de la feuille de surface, et la couche en voile cardé directement sur le coussin absorbant, on obtient un article absorbant selon l'invention ayant une douceur convenable due à la finesse des fibres utilisées pour la couche interne, une excellente vitesse de traversée par les liquides et qui ne peluche pas.

### Exemple 2

On répète l'exemple 1 en remplaçant les fibres de polyéthylène du voile cardé par des fibres bi-composants de même denier comprenant une âme de polyester et une enveloppe de polyéthylène.

On obtient un matériau non-tissé composite selon l'invention. Ce matériau présente un excellent temps de traversée par les liquides, une bonne résistance au remouillage et ne peluche pas lorsqu'il est utilisé comme voile de surface ou bande de zone d'entre-jambes dans un article d'hygiène, tel qu'une couche-culotte.

### Exemple 3

On répète l'exemple 1 en utilisant pour le voile cardé uniquement des fibres de polyester de 6 décitex. On enduit une face de ce voile cardé avec une émulsion aqueuse d'éthylène acétate de butyle (EBA), à raison de 4 g d'émulsion par mètre carré de voile. On lie ensuite le voile cardé par sa face enduite au voile de polyéthylène dans une calandre de contre-collage de type STORCK dont le cylindre chauffant est à une température de 120-150°C.

On obtient un matériau non-tissé composite selon l'invention.

### Exemple de réalisation d'un article d'hygiène absorbant selon l'invention.

On a représenté figures 2 et 3, à titre d'exemple, un article d'hygiène absorbant, tel qu'une couche-culotte, comportant une bande d'entre-jambes constituée d'un matériau non-tissé composite selon l'invention.

La couche-culotte représentée sur les figures 2 et 3 comprend de façon connue en soi, une feuille de support 10 imperméable aux liquides, un coussin absorbant 12, par exemple en pâte fluff de cellulose, éventuellement avec incorporation de matières polymères dites superabsorbantes, une bande de zone d'entre-jambes 27, une bande intermédiaire 29 de dimension analogue à celle de la bande de zone d'entre-jambes 27, disposée directement sur cette bande de zone d'entre-jambes, et généralement constituée d'un non-tissé classique (par exemple de type lié au filage), et une feuille de surface 13, par exemple un voile de non-tissé hydrophobe. Les deux feuilles 10 et 13 ont les mêmes dimensions et la même forme en sablier c'est-à-dire une forme rectangulaire avec deux échancrures latérales opposées délimitant, dans le sens de la longueur de la couche-culotte une zone 15 d'entre-jambes de largeur réduite entre deux zones d'extrémité 16 et 17 de largeur accrue. Le coussin absorbant 12 disposé entre les deux feuilles 10 et 13 présente également une forme en sablier mais de dimension plus faible que les feuilles 10 et 13 qui sont reliées entre-elles par exemple par collage sur tout le pourtour du coussin 12.

Des premiers éléments élastiques 18 longitudinaux, constitués par exemple chacun par un ou plusieurs brins ou fils élastiques ou par une bandelette élastique, sont fixés à l'état tendu à la feuille de support 10, au moins dans la zone d'entre-jambes 15 entre le fond des échancrures de la feuille 10 et le fond des échancrures correspondantes du coussin absorbant 12.

Par ailleurs, des attaches adhésives 19 sont fixées sur les bords latéraux opposés sur la zone d'extrémité 16 constituant la partie arrière de la couche-culotte.

La feuille de surface 13 comporte à sa partie médiane une découpe longitudinale médiane 25 pour former une ouverture, de préférence de forme oblongue. La largeur de l'ouverture est nettement inférieure à la largeur du coussin 12, de préférence inférieure à la moitié de la largeur du coussin 12 dans la zone d'entre-jambes. De plus, la feuille de surface 13 comporte, sur toute sa longueur, deux doubles plis en Z 30 vers le bas (vers le coussin 12) disposés de part et d'autre de l'ouverture 25, et des éléments élastiques 26 sont fixés à l'état tendu par collage dans les replis supérieurs de ces doubles plis 30, les deux replis de chaque double pli 30 étant solidarisés l'un à l'autre par collage ou tout autre moyen de façon à consolider ou rigidifier la feuille de couverture 13 à cet endroit et faciliter ainsi la découpe avec enlèvement de matière de l'ouverture 25, pratiquée ultérieurement. La bande d'entre-jambes 27 en matériau non-tissé composite selon l'invention est disposée directement au-dessus du coussin absorbant sur toute la longueur de la couche-culotte et a une largeur au moins égale à la largeur du coussin absorbant 12 dans la zone d'entre-jambes. La bande de zone d'entre-jambes 27 est liée au coussin absorbant 12 et/ou à la bande intermédiaire 29 par tout moyen approprié bien connu, et la bande intermédiaire 29, dans la réalisation représentée, est fixée à la feuille de surface 13, d'une part sur les deux bords transversaux de la couche-culotte et d'autre part suivant deux lignes longitudinales 28, par exemple des lignes de collage, situées dans une position légèrement décalée par rapport aux bords longitudinaux du coussin absorbant 12 dans la zone d'entre-jambes 15.

Bien qu'on ait représenté la feuille de surface comme comportant deux doubles plis en Z 30, il est également possible de modifier la forme de ces plis de toute manière appropriée, par exemple en réalisant des plis ayant la forme d'un S.

Egalement, les éléments élastiques 26 peuvent être disposés en tout endroit approprié autre que les replis supérieurs, par exemple les replis inférieurs.

La bande d'entre-jambes 27 est constituée par un matériau non-tissé composite selon l'invention comprenant une première couche d'un voile cardé à fibres partiellement désorientées et une deuxième couche d'un voile de non-tissé de type non-cardé, les première et seconde couches étant liées uniquement par thermofusion, la première couche étant en contact direct avec le coussin absorbant 12.

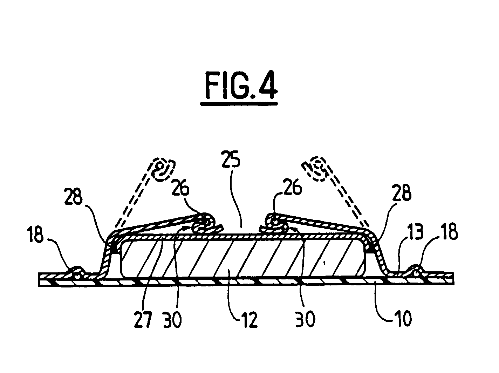

La figure 4 est une vue en coupe analogue à celle de la figure 3 d'une réalisation recommandée d'une couche-culotte selon l'invention. Cette réalisation est identique à celle des figures 2 et 3 excepté que l'on a supprimé le voile intermédiaire 29. La bande de zone d'entre-jambes 27 dans cette réalisation est disposée sur le coussin absorbant 12 avec la couche cardée en contact avec le coussin. La couche supérieure en voile de non-tissé de type non-cardé de la bande de zone d'entre-jambes est liée à la feuille de couverture suivant deux lignes longitudinales 28, par exemple des lignes de collage, analogues à celles de la figure 3. Dans cette réalisation, la couche en non-tissé de type non-cardé, qui se trouve directement en dessous de la découpe 25, sera directement en contact avec la peau de l'utilisateur.

Cette couche-culotte présente une vitesse élevée de traversée par les liquides, une bonne résistance au remouillage et ne peluche pas.

## Revendications

1. Matériau non-tissé composite caractérisé en ce qu'il comprend au moins une couche (2) formée d'un voile cardé de fibres thermoplastiques, hydrophobes lesdites fibres étant partiellement désorientées dans au moins une partie de l'épaisseur du voile pour qu'une partie desdites fibres ait une orientation angulaire par rapport au plan principal des fibres dudit voile, et une couche (3) formée d'un voile en non-tissé de type non-cardé, de fibres thermoplastiques, hydrophobes, les fibres d'au moins une des couches étant choisies parmi les fibres à bas point de fusion, les fibres bi-composants, les mélanges de telles fibres avec des fibres à point de fusion élevé et les fibres mélangées avec un liant, ou au moins une des couches est formée d'un voile dont une face est enduite d'un liant, les deux couches étant liées entre elles uniquement par thermofusion.

2. Matériau selon la revendication 1, caractérisé en ce que le taux de fibres désorientées dans la couche (2) de voile cardé est d'au moins 20 % en poids par rapport au poids total des fibres du voile.

3. Matériau selon la revendication 1 ou 2, caractérisé en ce que la couche de voile cardé a un grammage compris entre 20 et 60 g/m² et la couche de voile de non-tissé de type non-cardé a un grammage inférieur à 20 g/m².

4. Matériau selon l'une quelconque des revendications 1 à 3, caractérisé en ce que les fibres d'au moins une des couches sont constituées par un mélange de fibres de polyester, avec des fibres choisies parmi les fibres à bas point de fusion et les fibres bi-composants.

5. Matériau selon la revendication 4, caractérisé en ce que les fibres à bas point de fusion sont choisies parmi les fibres de polyéthylène, de polypropylène et de copolymères d'éthylène/propylène.

6. Matériau selon l'une quelconque des revendications 1 à 3, caractérisé en ce que les fibres mélangées avec un liant sont des fibres de polyester mélangées avec une colle réactivable à chaud ou une poudre à bas point de fusion.

7. Matériau selon l'une quelconque des revendications 1 à 5, caractérisé en ce que les fibres bi-composants, ou les fibres à bas point de fusion constituent 5 à 30 % en poids par rapport au poids total du voile.

8. Matériau selon l'une quelconque des revendications 1, 2, 3 ou 6, caractérisé en ce que la quantité de liant mélangé aux fibres ou déposé sur une face d'un desdits voiles est comprise entre 5 et 30 % en poids par rapport au poids total du voile.

9. Matériau selon la revendication 1, caractérisé en ce que le voile cardé est un voile composite comprenant au moins deux couches de voile cardé liées entre elles par thermofusion.

10. Procédé de fabrication d'un matériau non-tissé composite, caractérisé en ce qu'il comprend les étapes consistant à :
- former un voile cardé de fibres thermoplastiques, hydrophobes;
- désorienter partiellement les fibres du voile cardé pour obtenir dans au moins une partie de l'épaisseur du voile cardé une orientation angulaire des fibres par rapport au plan principal des fibres du voile cardé;
- former un voile en non-tissé de type non-cardé, de fibres thermoplastiques, hydrophobes;
- superposer le voile cardé à fibres partiellement désorientées et le voile en non-tissé de type non-cardé pour obtenir une première couche du voile cardé et une seconde couche du voile en non-tissé de type non-cardé, les fibres d'au moins une des couches étant choisies parmi les fibres à bas point de fusion, les fibres bi-composants, les mélanges de telles fibres avec des fibres à point de fusion élevé et les fibres mélangées à un liant; et
- lier les couches entre elles par thermofusion.

11. Procédé selon la revendication 10 caractérisé en ce que la liaison par thermofusion comprend une réactivation par air chaud traversant les couches.

12. Procédé de fabrication d'un matériau non-tissé composite, caractérisé en ce qu'il comprend les étapes consistant à :
- former un voile cardé de fibres thermoplastiques, hydrophobes;
- désorienter partiellement les fibres du voile cardé pour obtenir dans au moins une partie de l'épaisseur du voile cardé une orientation angulaire des fibres par rapport au plan principal des fibres du voile cardé;
- former un voile en non-tissé de type non-cardé, de fibres thermoplastiques, hydrophobes,
- enduire une des faces d'un desdits voiles avec un liant;
- superposer le voile cardé à fibres partiellement désorientées et le voile en non-tissé de type non-cardé pour obtenir une première couche du voile cardé et une deuxième couche du voile en non-tissé de type non-cardé de telle sorte que ladite face enduite de liant forme une interface entre lesdites couches; et
- lier les couches entre elles par thermofusion.

13. Procédé selon la revendication 12, caractérisé en ce que la liaison par thermofusion desdites couches s'effectue par calandrage des couches au moyen d'une calandre de contre-collage.

14. Procédé selon l'une quelconque des revendications 10 à 13, caractérisé en ce que l'étape de désorientation partielle des fibres du voile cardé comprend la désorientation angulaire, par rapport au plan principal des fibres, d'au moins 20 % en poids de fibres par rapport au poids total de fibres du voile.

15. Procédé selon l'une quelconque des revendications 13 à 14, caractérisé en ce que l'angle de désorientation des fibres par rapport au plan principal des fibres est d'au moins 35°.

16. Procédé selon l'une quelconque des revendications 10 à 15, caractérisé en ce que l'étape de formation du voile cardé et l'étape de désorientation partielle des fibres dudit voile s'effectuent conjointement au moyen d'une pseudo-carde.

17. Article d'hygiène absorbant, par exemple une couche-culotte, comprenant une couche externe imperméable aux liquides corporels (10), un coussin absorbant (12) perméable aux liquides corporels fixé à la couche externe, la couche externe (10) et le coussin absorbant (12) comportant des parties d'extrémité (16, 17) élargies réunies par une zone d'entre-jambes (15) de plus faible largeur, une feuille de surface (13), de préférence hydrophobe, et une bande de zone d'entre-jambes perméable aux fluides corporels (27) disposée entre le coussin absorbant (12) et la feuille de surface (13) et de largeur analogue à la largeur du coussin dans la zone d'entre-jambes et de longueur au moins égale à la longueur du coussin et liée au coussin, caractérisé en ce que la bande de zone d'entre-jambes est constituée du matériau composite selon l'une quelconque des revendications 1 à 9, la couche en voile cardé étant directement en contact avec le coussin absorbant.

18. Article d'hygiène absorbant selon la revendication 17 dans lequel la feuille de surface (13) comporte une découpe longitudinale médiane (25) formant une ouverture, de préférence de forme oblongue.

19. Article d'hygiène absorbant selon la revendication 17 ou 18, comportant en outre une bande intermédiaire (29) en non-tissé de dimension analogue à la bande de zone d'entre-jambes et disposée directement sur la bande de zone d'entre-jambes.

## Patentansprüche

1. Vliesstoff-Verbundmaterial,
dadurch **gekennzeichnet**, daß
es mindestens eine Schicht (2), die aus einem kardierten Schleierstoff aus hydrophoben thermoplastischen Fasern gebildet ist, wobei die genannten Fasern in mindestens einem Teilbereich der Dicke der Schleierstoffbahn teilweise desorientiert sind, damit ein Teil der genannten Fasern eine gewinkelte Orientierung aufweist, bezogen auf den grundsätzlichen Aufbauplan der Fasern der genannten Schleierstoffbahn, sowie eine Schicht (3) aus einem Schleierstoff aus Vliesstoff vom nicht-kardierten Typ aus hydrophoben thermoplastischen Fasern umfaßt, wobei die Fasern mindestens einer der Schichten aus niedrig schmelzenden Fasern, Bikomponentenfasern, Mischungen derartiger Fasern mit höherschmelzenden Fasern und aus mit einem Bindemittel vermischten Fasern ausgewählt sind, oder wobei mindestens eine der Schichten aus einer Schleierstoffbahn gebildet ist, deren eine Seite mit einem Bindemittel überzogen ist, wobei die beiden Schichten untereinander alleiniglich durch Thermofusion verbunden ist.

2. Material gemäß Anspruch 1,
dadurch **gekennzeichnet**, daß
die Gehaltsmenge an desorientierten Fasern in der Schicht (2) aus kardiertem Schleierstoff mindestens 20 Gew.% ausmacht, bezogen auf das Gesamtgewicht der Fasern der Schleierstoffbahn.

3. Material gemäß Anspruch 1 oder 2,
dadurch **gekennzeichnet**, daß
die Schicht aus kardiertem Schleierstoff ein Grammgewicht von 20 bis 60 g/m² und die Schicht aus Schleierstoff aus Vliesstoff vom nicht-kardierten Typ ein Grammgewicht von weniger als 20 g/m² aufweisen.

4. Material gemäß jedem der Ansprüche 1 bis 3,
dadurch **gekennzeichnet**, daß
die Fasern mindestens einer der Schichten aus einer Mischung aus Polyesterfasern mit Fasern zusammengesetzt sind, die aus niedrigschmelzenden Fasern und aus Bikomponentenfasern ausgewählt sind.

5. Material gemäß Anspruch 4,
dadurch **gekennzeichnet**, daß
die niedrigschmelzenden Fasern aus Fasern aus Faern aus Polyethylen, Polypropylen und aus Copolymeren von Ethylen/Propylen ausgewählt sind.

6. Material gemäß jedem der Ansprüche 1 bis 3,
dadurch **gekennzeichnet**, daß
die mit einem Bindemittel vermischten Fasern Polyesterfasern sind, die mit einem wärmeaktivierbaren Klebstoff oder einem niedrigschmelzenden Pulver vermischt sind.

7. Material gemäß jedem der Ansprüche 1 bis 5,
dadurch **gekennzeichnet**, daß
die Bikomponentenfasern oder die niedrigschmelzenden Fasern 5 bis 30 Gew.% ausmachen, bezogen auf das Gesamtgewicht des Schleierstoffs.

8. Material gemäß jedem der Ansprüche 1, 2, 3 oder 6,
dadurch **gekennzeichnet**, daß
die Menge an Bindemittel, die mit den Fasern vermischt oder auf eine Seite einer der genannten Schleierstoffbahnen aufgebracht ist, 5 bis 30 Gew.% beträgt, bezogen auf das Gesamtgewicht des Schleierstoffs.

9. Material gemäß Anspruch 1,
dadurch **gekennzeichnet**, daß
der kardierte Schleierstoff ein Verbundschleierstoff ist, umfassend mindestens zwei Schichten aus kardiertem Schleierstoff, die miteinander durch Thermofusion verbunden sind.

10. Verfahren zur Herstellung eines Vliesstoff-Verbundmaterials,
dadurch **gekennzeichnet**, daß
es Stufen umfaßt, in denen man:
- einen kardierten Schleierstoff aus hydrophoben thermoplastischen Fasern bildet;
- die Fasern des kardierten Schleierstoffs teilweise desorientiert, um in mindestens einem Teilbereich der Dicke der kardierten Schleierstoffbahn eine gewinkelte Orientierung der Fasern zu erhalten, bezogen auf den grundsätzlichen Aufbauplan der Fasern der kardierten Schleierstoffbahn;
- einen Schleierstoff aus Vliesstoff vom nicht-kardierten Typ aus hydrophoben thermoplastischen Fasern bildet;
- die kardierte Schleierstoffbahn aus teilweise desorientierten Fasern und die Schleierstoffbahn aus Vliesstoff vom nicht-kardierten Typ übereinanderlegt, um eine erste Schicht aus kardiertem Schleierstoff und eine zweite Schicht des Schleierstoffs aus Vliesstoff vom nicht-kardierten Typ zu erhalten, wobei die Fasern mindestens einer der Schichten aus niedrigschmelzenden Fasern, Bikomponentenfasern, Mischungen derartiger Fasern mit höherschmelzenden Fasern und aus mit einem Bindemitel vermischten Fasern ausgewählt sind; und
- die Schichten untereinander durch Thermoschmelzen verbindet.

11. Verfahren gemäß Anspruch 10,
dadurch **gekennzeichnet**, daß
das Verbinden durch Thermoschmelzen eine Aktivierung mit heißer Luft umfaßt, die durch die Schichten geleitet wird.

12. Verfahren zur Herstellung eines Vliesstoff-Verbundmaterials,
dadurch **gekennzeichnet**, daß
es Stufen umfaßt, in denen man:
- einen kardierten Schleierstoff aus hydrophoben thermoplastischen Fasern bildet;
- die Fasern des kardierten Schleierstoffs teilweise desorientiert, um in mindestens einem Teilbereich der Dicke der kardierten Schleierstoffbahn eine gewinkelte Orientierung der Fasern zu erhalten, bezogen auf den grundsätzlichen Aufbauplan der Fasern der kardierten Schleierstoffbahn,
- einen Schleierstof aus Vliesstoff vom nicht-kardierten Typ aus hydrophoben thermoplastischen Fasern bildet;
- eine der Seiten einer der genannten Schleierstoffbahnen mit einem Bindemittel überzieht;
- die kardierte Schleierstoffbahn aus teilweise desorientierten Fasern und die Schleierstoffbahn aus Vliesstoff vom nicht-kardierten Typ übereinanderlegt, um eine erste Schicht des kardierten Schleierstoffs und eine zweite Schicht des Schleierstoffs aus Vliesstoff vom nicht-kardierten Typ in einer solchen Weise zu erhalten, daß die genannte, mit dem Bindemittel überzogene Seite eine Grenzfläche zwischen den genannten Schichten bildet; und
- die Schichten untereinander durch Thermoschmelzen verbindet.

13. Verfahren gemäß Anspruch 12,
dadurch **gekennzeichnet**, daß
das Verbinden durch Thermoschmelzen der genannten Schichten durch Kalandern der Schichten mittels eines Kalanders unter Gegenverklebung erfolgt und durchgeführt wird.

14. Verfahren gemäß jedem der Ansprüche 1^0 bis 13,
dadurch **gekennzeichnet**, daß
die Stufe der teilweisen Desorientierung der Fasern der kardierten Schleierstoffbahn eine Maßnahme zur gewinkelten Desorientierung umfaßt, bezogen auf den grundsätzlichen Aufbauplan der Fasern, und zwar bei mindestens 20 Gew.% an Fasern, bezogen auf das Gesamtgewicht der Fasern der Schleierstoffbahn.

15. Verfahren gemäß jedem der Ansprüche 13 und 14,
dadurch **gekennzeichnet**, daß
der Desorientierungswinkel der Fasern, bezogen auf den grundästzlichen Aufbauplan der Fasern, mindestens 35° beträgt.

16. Verfahren gemäß jedem der Ansprüche 10 bis 15,
dadurch **gekennzeichnet**, daß
die Stufe zur Bildung der kardierten Schleierstoffbahn und die Stufe zur teilweisen Desorientierung der Fasern der genannten Schleierstoffbahn mittels eines Pseudo-Kard gemeinsam durchgeführt werden.

17. Absorbierender Hygieneartikel, beispielsweise Windel-Höschen, umfassend eine für Körperflüssigkeiten undurchlässige Außenschicht (10), ein für Körperflüssigkeiten durchlässiges absorbierendes Kissen (12), das an die Außenschicht fixiert ist, wobei die Außenschicht (10) und das absorbierende Kissen (12) vergrößerte Außenteilstücke (16, 17) aufweisen, die mit einer Zwischenbeinzone (15) geringerer Größe in Verbindung stehen, umfassend ferner eine vorzugsweise hydrophobe Oberflächenfolie (13) sowie eine für Körperflüssigkeiten durchlässige Binde (27) für die Zwischenbeinzone, welche zwischen dem absorbierenden Kissen (12) und der Oberflächenfolie (13) angeordnet ist, eine Größe, die der Größe des Kissens in der Zwischenbeinzone gleicht, und eine Länge aufweist, die mindestens so lang wie das Kissen ist, und welche mit dem Kissen verbunden ist,
dadurch **gekennzeichnet**, daß
die Binde für die Zwischenbeinzone aus dem Verbundmaterial gemäß jedem der Ansprüche 1 bis 9 zusammengesetzt ist, wobei die Schicht aus kardiertem Schleierstoff direkt in Kontakt mit dem absorbierenden Kissen vorliegt.

18. Absorbierender Hygieneartikel gemäß Anspruch 17, worin die Oberflächenfolie (13) einen longitudinalen mittleren Ausschnitt (25) aufweist, der eine Öffnung bildet, vorzugsweise in länglicher Form.

19. Absorbierender Hygieneartikel gemäß Anspruch 17 oder 18, welcher außerdem eine Zwischenbinde (29) aus Vliesstoff in einer Abmessung, die der binde für die Zwischenbeinzone gleicht, aufweist, die direkt über der Binde für die Zwischenbeinzone angeordnet ist.

## Claims

1. Composite nonwoven material characterized in that it comprises at least one layer (2) made of a carded voile of hydrophobic thermoplastic fibers, the said fibers being partially disoriented in at least a proportion of the thickness of the voile so that a proportion of the said fibers has an angular orientation in relation to the main plane of the fibers of the said voile, and a layer (3) made of a voile of nonwoven of noncarded type, of hydrophobic, thermoplastic fibers, the fibers of at least one of the layers being chosen from fibers with a low melting point, two-component fibers, mixtures of such fibers with fibers of high melting point and fibers mixed with a binder, or at least one of the layers is made of a voile one face of which is coated with a binder, the two layers being bonded to each other solely by heat-melting.

2. Material according to claim 1,characterized in that the proportion of disoriented fibers in the layer (2) of carded voile is at least 20 % by weight relative to the total weight of the fibers of the voile.

3. Material according to claim 1 or 2, characterized in that the layer of carded voile has a weight per unit area of between 20 and 60 g/m² and the layer of voile of nonwoven of noncarded type has a weight per unit area lower than 20 g/m².

4. Material according to any one of claims 1 to 3, characterized in that the fibers of at least one of the layers consist of a mixture of polyester fibers with fibers chosen from fibers with a low melting point and two-component fibers.

5. Material according to claim 4, characterized in that the fibers with a low melting point are chosen from polyethylene, polypropylene and ethylene/propylene copolymer fibers.

6. Material according to any one of claims 1 to 3, characterized in that the fibers mixed with a binder are polyester fibers mixed with a heat-reactivable adhesive or a powder with a low melting point.

7. Material according to any one of claims 1 to 5, characterized in that the two-component fibers or the fibers with a low melting point constitute 5 to 30 % by weight relative to the total weight of the voile.

8. Material according to any one of claims 1, 2, 3 or 6, characterized in that the quantity of binder mixed with the fibers or deposited on one face of one of the said voiles is between 5 and 30 % by weight relative to the total weight of the voile.

9. Material according to claim 1, characterized in that the carded voile is a composite voile comprising at least two layers of carded voile bonded together by heat-melting.

10. Process for the manufacture of a composite nonwoven material, characterized in that it comprises the stages consisting in:
- forming a carded voile of hydrophobic thermoplastic fibers;
- partially disorienting the fibers of the carded voile to obtain in at least a proportion of the thickness of the carded voile an angular orientation of the fibers in relation to the main plane of the fibers of the carded voile;
- forming a voile made of nonwoven of noncarded type, of hydrophobic thermoplastic fibers;
- superposing the carded voile with partially disoriented fibers and the voile made of nonwoven or noncarded type to obtain a first layer of carded voile and a second layer of voile made of nonwoven of noncarded type, the fibers of at least one of the layers being chosen from fibers with a low melting point, two-component fibers, mixtures of such fibers with fibers of high melting point and fibers mixed with a binder; and
- bonding the layers together by heat-melting.

11. Process according to claim 10, characterized in that the bonding by heat-melting comprises a reactivation by hot air passing through the layers.

12. Process for the manufacture of a composite nonwoven material, characterized in that it comprises the stages consisting in:
- forming a carded voile of hydrophobic thermoplastic fibers;
- partially disorienting the fibers of the carded voile to obtain in at least a proportion of the thickness of the carded voile an angular orientation of the fibers in relation to the main plane of the fibers of the carded voile;
- forming a voile made of nonwoven of noncarded type, of hydrophobic thermoplastic fibers,
- coating one of the faces of one of the said voiles with a binder;
- superposing the carded voile with partially disoriented fibers and the voile made of nonwoven of noncarded type to obtain a first layer of the carded voile and a second layer of the voile made of nonwoven of noncarded type so that the said binder-coated face forms an interface between the said layers; and
- bonding the layers to each other by heat-melting.

13. Process according to claim 12, characterized in that the bonding of the said layers by heat-melting is carried out by calendering the layers by means of an adhesive-backing calender.

14. Process according to any one of claims 10 to 13, characterized in that the stage of partial disorientation of the fibers of the carded voile comprises the angular disorientation, in relation to the main plane of the fibers, of at least 20 % by weight of fibers in relation to the total weight of the fibers of the voile.

15. Process according to either of claims 13 to 14, characterized in that the angle of disorientation of the fibers in relation to the main plane of the fibers is at least 35°.

16. Process according to any one of claims 10 to 15, characterized in that the stage of formation of the carded voile and the stage of partial disorientation of the fibers of the said voile are carried out jointly by means of a pseudocard.

17. Absorbent article of hygiene, for example a diaper, comprising an outer layer which is impervious to body fluids (10), an absorbent pad (12) which is permeable to body fluids and attached to the outer layer, the outer layer (10) and the absorbent pad (12) comprising widened end parts (16, 17) joined by a crotch region (15) of smaller width, a preferably hydrophobic surface sheet (33), and a crotch region strip which is permeable to body fluids (27) and arranged between the absorbent pad (12) and the surface sheet (13) and of width similar to the width of the pad in the crotch region and of length which is at least equal to the length of the pad and bonded to the pad, characterized in that the crotch region strip consists of the composite material according to any one of claims 1 to 9, the layer made of carded voile being directly in contact with the absorbent pad.

18. Absorbent article of hygiene according to claim 17, in which the surface sheet (13) comprises a medium lengthwise cutout (25) forming an opening, preferably of oblong shape.

19. Absorbent article of hygiene according to claim 17 or 18, additionally comprising an intermediate strip (29) of nonwoven, similar in size to the crotch region strip and arranged directly on the crotch region strip.
